Europäisches Patentamt

(19) European Patent Office

Office européen des brevets

(11) Publication number : **0 500 229 A1**

(12) **EUROPEAN PATENT APPLICATION**

(21) Application number : 92300917.9

(22) Date of filing : 04.02.92

(51) Int. Cl.⁵ : **B05D 1/32,** A61B 17/06, C23C 22/00, C09D 5/00

(30) Priority : 21.02.91 JP 47304/91

(43) Date of publication of application :
26.08.92 Bulletin 92/35

(84) Designated Contracting States :
DE FR GB

(71) Applicant : MATSUTANI SEISAKUSHO CO. LTD.,
743 Oaza Naka-Akutsu
Takanezawa-Machi Shioya-Gun Tochigi-ken (JP)

(72) Inventor : Matsutani, Masaaki
740 Oaza Nakaakutsu, Takanezawa-Machi Shioya-Gun, Tochigi-Ken (JP)

(74) Representative : Votier, Sidney David et al
CARPMAELS & RANSFORD 43, Bloomsbury Square
London WC1A 2RA (GB)

(54) **Suture needle coating method.**

(57) There is disclosed a method of coating a metallic suture needle (1), having a blind hole (1b), with silicone. First, a silicone solution (5) containing silicone and a solvent is deposited on an outer surface of the suture needle (1) over an entire length thereof to form a silicone layer. At this time, the silicone solution (5) is inevitably supplied into the blind hole (1b) of the suture needle (1). The suture needle (1) is placed in a gas atmosphere, so that a silicone coating layer (5a), which is bonded to the outer surface of the suture needle (1) with a high strength and is difficult to be dissolved in a solvent, is formed in the innermost portion of the silicone layer. The silicone coating layer (5a) is not formed on at least part of the inner peripheral surface of the blind hole (1b). Finally, the silicone layer (5b) except for the silicone coating layer (5a) is removed by the use of a solvent. As a result, the silicone coating layer (5a) remains at the outer surface of the suture needle (1), and the metallic surface of the suture needle (1) is exposed at at least part of the inner peripheral surface of the blind hole (1b).

Fig.3

EP 0 500 229 A1

BACKGROUND OF THE INVENTION

This invention relates to a method of coating a suture needle with silicone.

A suture needle is required to have a good ability of piercing into a living tissue. To meet this requirement, Japanese Laid-Open Patent Application No. 62-101236 proposes a technique of coating at least a distal end portion of a suture needle with silicone. Japanese Laid-Open Utility Model Application No. 63-26309 discloses a technique of silicone-coating an outer peripheral surface of a suture needle over the entire length thereof except for its distal end portion. U. S. Patent Nos. 4,905,695 and 4,959,068 disclose a technique of silicone-coating a suture needle. Particularly, the above Japanese Laid-Open Patent Application No. 62-101236 discloses a method in which the suture needle is silicone-coated by dipping it in a silicone solution.

In the above-mentioned Japanese applications and U. S. patents, for example, when part of the suture needle is silicone-coated by dipping, the productivity is not good. The reason for this is that the dipping must be carried out while grasping that portion of the suture needle which is not to be coated with silicone, and therefore a number of suture needles can not be dipped at a time. On the other hand, when the whole of the suture needle is silicone-coated, for example, by dipping, the suture needles do not need to be held independently of one another, and therefore a number of suture needles can be dipped at a time, which results in a high productivity. In this case, however, the silicone coating is applied even to an inner surface of a gut-mounting hole in the suture needle, which invites the possibility that the gut may not be satisfactorily fixed to the gut-mounting hole. Particularly when one end portion of the gut is inserted into a blind hole formed axially in the proximal end portion of the suture needle, and then is fixed to this blind hole by compressively deforming the proximal end portion of the suture needle, the fixing of the gut to the blind hole can not be effected satisfactorily, though this fixing is important.

Further, in the above-mentioned Japanese applications and U. S. patents, the silicone layer coated on the suture needle is formed merely by volatilizing a solvent from the silicone solution, and therefore the strength of bonding between the silicone layer and the outer surface of the suture needle is weak. As a result, when the suture needle pierces into a living tissue, the silicone layer can be easily peeled, and a piercing resistance is much increased with the increase of the frequency of piercing.

SUMMARY OF THE INVENTION

It is an object of this invention to provide a suture needle coating method which can form a silicone coating layer less liable to increase a piercing resistance with the increase of the frequency of piercing of the suture needle into a living tissue, and ensures a positive fixing of a gut to a blind hole in the suture needle, and provides a high productivity.

According to the present invention, there is provided a suture needle coating method comprising the steps of:

(a) providing a suture needle of metal having a pointed distal end and a blind hole formed axially in a proximal end portion of the suture needle, and depositing a silicone solution on an outer surface of the suture needle over an entire length thereof to form a silicone layer, and supplying the silicone solution also to the blind hole, the silicone solution containing silicone and a solvent;

(b) placing the suture needle in a gas atmosphere, so that molecules of the silicone adjacent to the outer surface of the suture needle are bonded to the metal of the outer surface of the suture needle with the aid of molecules of the gas passing through the silicone layer, thereby forming a silicone coating layer in an innermost portion of the silicone layer except for at least part of an inner peripheral surface of the blind hole, the silicone coating layer being difficult to be dissolved in a solvent, the remainder of the silicone layer disposed outwardly of the silicone coating layer being soluble in a solvent; and

(c) removing the remainder of the silicone layer by the use of a solvent, so that the silicone coating layer remains at the outer surface of the suture needle, and so that the metallic surface of the suture needle is exposed at at least part of the inner peripheral surface of the blind hole.

BRIEF DESCRIPTION OF THE DRAWINGS

Fig. 1 is a perspective view of a suture needle used in a method of the present invention;

Fig. 2 is an enlarged cross-sectional view of a proximal end portion of the suture needle on which a silicone solution deposits;

Fig. 3 is an enlarged cross-sectional view of the proximal end portion of the suture needle, showing the condition of a silicone layer after a heat treatment;

Fig. 4 is an enlarged cross-sectional view of the proximal end portion of the suture needle, showing a condition in which the silicone layer except for a silicone coating layer is removed; and

Figs. 5 and 6 are views similar to Fig. 3, but showing modified forms of the invention, respectively.

DESCRIPTION OF THE PREFERRED EMBODIMENTS

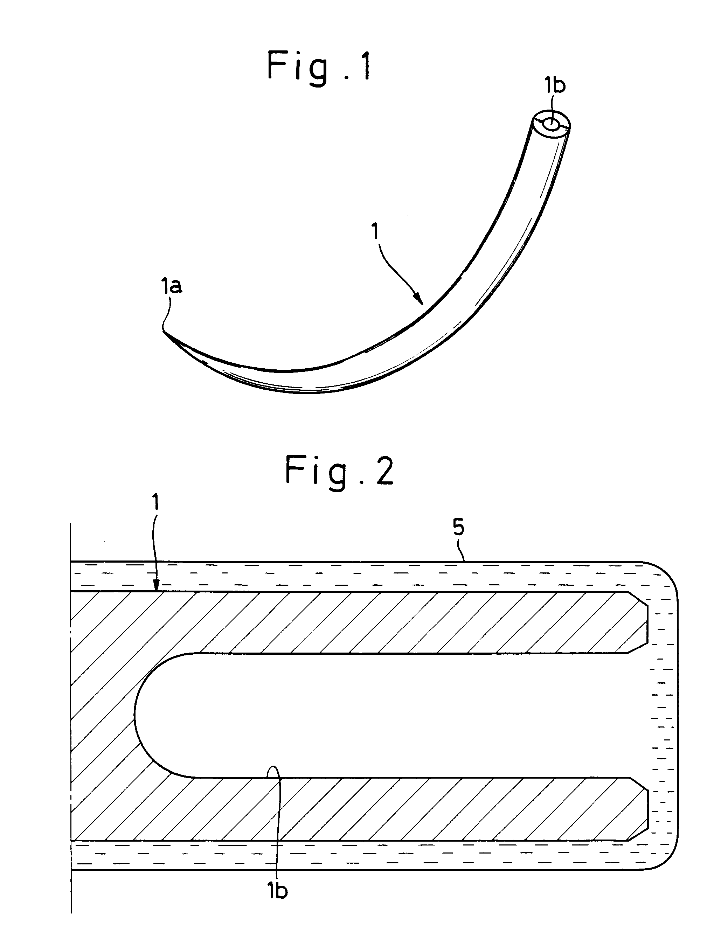

A method of the present invention will now be described in detail. First, there is prepared a suture needle 1 of the taper-point type as shown in Fig. 1. The suture needle 1 is formed by cold drawing a wire, for example, of austenite-type stainless steel, and then by cutting this drawn wire into a predetermined length, and then by processing the cut wire by a well known method. The suture needle has a curved configuration, and has a circular cross-section throughout the length thereof. The distal end portion of the suture needle 1 is tapered, and has a point 1a at its distal end. A blind hole 1b is formed in the proximal end portion of the suture needle 1, and extends in the direction of the axis thereof. The prepared suture needle 1 is already subjected to chemical polishing or electropolishing and subsequently to washing and drying.

The suture needle 1 is dipped in a silicone solution. The silicone solution consists of about 1 wt.% to about 50 wt.% (for example, 10 wt.%) silicone and the balance solvent. As the silicone, for example, 360 MEDICAL FLUID (tradename of Dowcorning Co., Ltd.) consisting of dimethyl polysiloxane is used. As the solvent, for example, acetone, fleon, trichloroethane or methylene chloride is used. A number of (for example, two to three thousands) suture needles 1 are put into a net-like container of stainless steel, and are dipped in the above silicone solution. By this dipping, the silicone solution covers the entire outer peripheral surface of each suture needle 1, and also covers the proximal end surface thereof. Since the silicone solution is low in viscosity, this solution is also filled in the blind hole 1b. After the dipping, the above container is taken up from the silicone solution bath. The condition of deposition of the silicone solution 5 on the suture needle 1 is shown in Fig. 2. Namely, a silicone layer of the silicone solution 5 deposits on the outer peripheral surface of the suture needle 1 over the entire length thereof from its proximal end to the point 1a, and also deposits on the proximal end surface thereof, and the silicone solution is also kept filled in the blind hole 1b.

The solvent of the silicone solution 5 deposited on the suture needle 1 is caused to volatilize almost completely by leaving the suture needle 1 after the dipping for about 10 minutes, and only the silicone layer consisting essentially of the silicone remains. However, it is thought that part of the solvent remains unvolatilized at the inner end of the blind hole 1b.

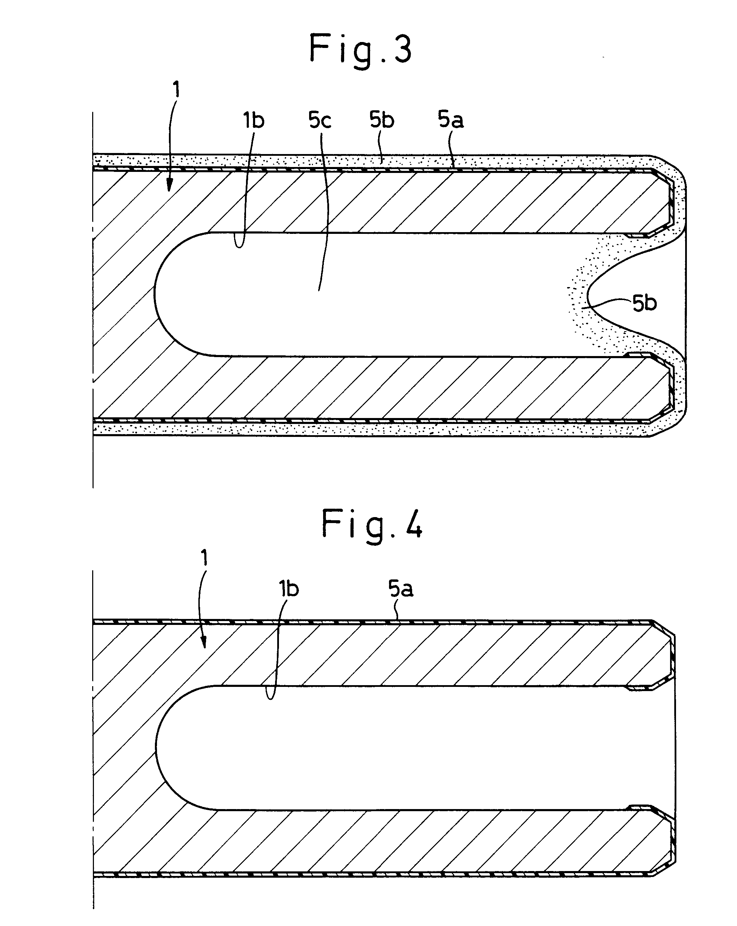

Next, the net-like container holding a number of coated suture needles 1 is introduced into a furnace, and is heated in an atmosphere of the air at 250 to 400°C for 1 to 10 hours (preferably, at 300 to 350°C for about 5 hours). During this heat treatment step, nitrogen ($N_2$) molecules and oxygen ($O_2$) molecules in the air within the furnace pass through the silicone layer to reach the outer surface of each suture needle 1, and assist in bonding the metal atoms on this outer surface with the silicone molecules. By this bonding, a silicone coating layer 5a is formed on the outer surface of the suture needle 1, as shown in Fig. 3. This phenomenon has not yet been theoretically proven. In the silicone used in this embodiment, the above bonding can be obtained by heating. The silicone coating layer 5a is very thin, and has a thickness generally on the order of a silicone molecule. The strength of bonding of the silicone coating layer 5a to the outer surface of the suture needle 1 is high. In this heat treatment step, the silicone layer is so changed as to include the above silicone coating layer 5a and a silicone cover layer 5b formed on the outside of the silicone coating layer 5a. The silicone cover layer 5b is higher in viscosity than the silicone layer before the heating. The silicone cover layer 5b is soluble by a solvent, but the silicone coating layer 5a is difficult to be dissolved by such a solvent since the strength of bonding of the silicone coating layer 5a to the outer surface of the suture needle 1 is high.

During the above heat treatment step, that portion of the silicone layer in the blind hole 1b which is disposed in the vicinity of the open outer end of this hole 1b is constituted by the silicone cover layer 5a of a high viscosity, and the other portion (i.e., that portion of the silicone layer disposed in the inner side of the blind hole 1b) remains as a generally liquid-state silicone 5c generally equal in viscosity to the silicone (starting material). The solvent may slightly remain in that portion of the silicone 5c disposed in the deepest portion of the blind hole 1b. The amounts of the nitrogen molecules and the oxygen molecules passing through the silicone layer within the blind hole 1b are much smaller than the amounts of these molecules passing through the silicone layer formed on the outer surface of the suture needle 1, and therefore any silicone coating layer of a high bonding strength is not formed in the inner surface of the blind hole 1b except for that portion in the vicinity of the open outer end of the blind hole 1b. In other words, during the above heat treatment step, the silicone coating layer 5a is formed only on the outer surface of the suture needle 1, but is not substantially formed on the inner surface of the blind hole 1b.

Preferably, during the above heat treatment step, a blast of hot air is applied to the suture needles 1, using a fan. By doing so, a fresh supply of the air is always given to the silicone layer on the outer surface of each suture needle, thereby promoting the formation of the silicone coating layer 5a.

Next, a number of suture needles 1, which are in the cordition shown in Fig. 3 and are held in the net-like container, are dipped, together with this container, in a solvent for 3 to 4 hours. As this solvent, for example, acetone, fleon, trichloroethane, or methylene chloride is used. By this dipping, the silicone cover layer 5b, formed on the outer periphery of the suture needle 1 over the entire length thereof and on the proximal end surface thereof, is dissolved in the solvent, and is removed from the suture needle 1.

Also, the silicone cover layer 5b and the silicone 5 which are disposed within the blind hole 1b are dissolved in the solvent, and are removed from the blind hole 1b. As a result, as shown in Fig. 4, only the silicone coating layer 5a formed on the outer periphery of the suture needle 1 over the entire length there and on the proximal end surface thereof remains, and the surface of the stainless steel is exposed at the inner surface of the blind hole 1b. Thereafter, the suture needles 1 are left, so that the solvent volatilizes.

Thus, the coating of the suture needles 1 is finished. With this coating method, since a number of suture needles 1 can be treated at a time, the productivity is very high. One end portion of a gut is inserted into the blind hole 1b of the suture needle 1, and then the proximal end portion of the suture needle 1 is compressively deformed, thereby fixedly securing the gut to the suture needle 1. Since the silicone coating layer 5a of a small frictional resistance is not formed on the inner surface of the blind hole 1b, a large frictional resistance is provided between the inner peripheral surface of the blind hole 1b and the end portion of the gut, and therefore the gut is firmly retained.

When the above suture needle 1 with the gut is actually used for suturing purposes, the friction between the suture needle and a living tissue is small since the silicone coating layer 5a is formed on the outer peripheral surface of the suture needle 1, and therefore an enhanced piercing ability of the suture needle 1 is obtained. Further, since the strength of bonding of the silicone coating layer 5a to the outer surface of the suture needle 1 is high, the silicone coating layer 5a is less liable to be peeled when the suture needle 1 is pierced into the living tissue. Therefore, the rate of increase of the frictional resistance between the suture needle 1 and the living tissue is restrained when the piercing is repeated.

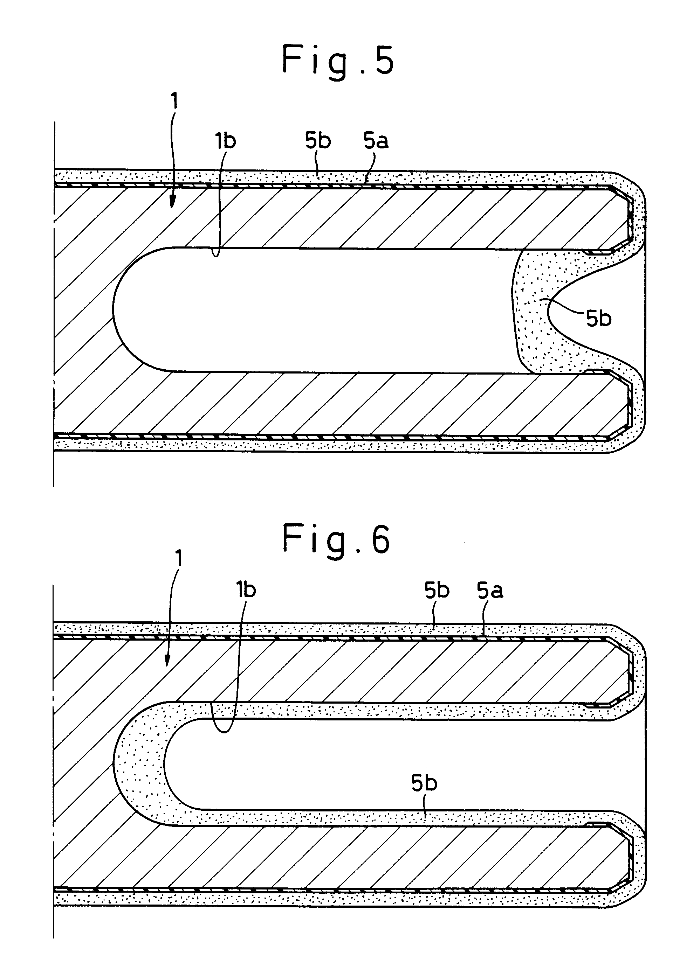

There is a possibility that when the suture needle 1 is dipped in the silicone solution, the air may be entrapped in the blind hole 1b, in which case the silicone solution is not fully filled in the blind hole 1b, and merely the open outer end of the blind hole 1b is closed by the silicone solution. In this case, as shown in Fig. 5, a silicone layer, resulting from the silicone solution through the volatilization of the solvent, remains as a silicone cover layer 5b of a high viscosity, and the surface of the stainless steel remains exposed at the inner surface of the blind hole 1b. Therefore, if this silicone cover layer 5b is removed by a solvent, the same condition as shown in Fig. 4 can be obtained. Those portions of Fig. 5 corresponding to those of Fig. 3 are designated by identical reference numerals, respectively, and explanation thereof are omitted.

In the case where the blind hole 1b has a large diameter, the silicone solution deposits on the entire inner peripheral surface and the inner end surface (the bottom) of the blind hole 1b when the suture nee-

dle 1 is dipped in the silicone solution, so that a space communicating with the exterior is formed in the blind hole 1b. In this case, also, by adjusting the heat treatment time, a silicone coating layer 5a of a high bonding strength is formed on the outer surface of the suture needle 1 whereas any silicone coating layer is not formed on the inner peripheral surface and the inner end surface of the blind hole 1b, and only a silicone cover layer 5b of a high viscosity is formed on these inner surfaces, as shown in Fig. 6. The reason for this is that nitrogen and oxygen are not sufficiently supplied to the interface between the inner surfaces of the blind hole 1b and the silicone layer because of a poor circulation of the air in the blind hole 1b.

The present invention is not limited to the above embodiments, and various modifications can be made. For example, the present invention can be applied to a suture needle of the cutting edge type having a distal end portion of a polygonal cross-section.

Supersonic vibration may be applied to the silicone solution when the suture needles are to be dipped in the silicone solution. By doing so, the air is prevented from being entrapped in the blind hole, and the silicone solution can be filled in the blind hole.

Supersonic vibration may be applied to the solvent when the silicone cover layer formed on the suture needle is to be dissolved by the solvent. By doing so, the solvent can be positively supplied into the blind hole to remove the silicone layer in the blind hole.

The silicone solution may be deposited on the suture needle by spraying the silicone solution to the suture needle. The solvent may be sprayed to the suture needle so as to remove, from the suture needle, the silicone layer except for the silicone coating layer.

After the suture needles are dipped in the silicone solution, the suture needles may not be left for drying, and instead may be introduced into the heating furnace. In this case, the drying of the silicone solution is effected at the initial stage of the heat treatment.

The suture needle having the silicone layer deposited thereon may be placed in the interior of the heating furnace which is in an atmosphere substantially consisting of nitrogen, so as to form the silicone coating layer of a high bonding strength. In this case, it is effective to apply a fresh supply of nitrogen gas by the use of a fan.

In the case where a solution containing a special type of silicone is used, the silicone coating layer bonded to the outer surface of the suture needle with a high strength, as well as the silicone cover layer on the outside of this silicone coating layer, can be formed merely by leaving the suture needle in the atmosphere at room temperature.

## Claims

1. A suture needle coating method comprising the step of :

(a) providing a suture needle (1) of metal having a pointed distal end (1a) and a blind hole (1b) formed axially in a proximal end portion of said suture needle, and depositing a silicone solution (5) on an outer surface of said suture needle over an entire length thereof to form a silicone layer, and supplying said silicone solution also to said blind hole, said silicone solution containing silicone and a solvent;

CHARACTERIZED by the steps of :

(b) placing said suture needle (1) in a gas atmosphere, so that molecules of the silicone adjacent to the outer surface of said suture needle are bonded to the metal of the outer surface of said suture needle with the aid of molecules of the gas passing through said silicone layer, thereby forming a silicone coating layer (5a) in an innermost portion of said silicone layer except for at least part of an inner peripheral surface of said blind hole (1b), said silicone coating layer being difficult to be dissolved in a solvent, the remainder (5b) of said silicone layer disposed outwardly of said silicone coating layer being soluble in a solvent; and

(c) removing said remainder of said silicone layer by the use of a solvent, so that said silicone coating layer remains at the outer surface of said suture needle, and so that the metallic surface of said suture needle is exposed at at least part of the inner peripheral surface of said blind hole.

2. A method according to claim 1, in which said silicone solution (5) is deposited on the outer surface of said suture needle (1) by dipping said suture needle in said silicone solution.

3. A method according to claim 2, in which said silicone solution (5) is filled in said blind hole (1b) of said suture needle (1) in said dipping operation.

4. A method according to claim 1, in which said silicone solution (5) is deposited on the outer surface of said suture needle (1) by spraying said silicone solution to said suture needle.

5. A method according to claim 1, in which in said step (b), said suture needle (1) is heated.

6. A method according to claim 1, in which said step (b) is carried out in an atmosphere of the air.

7. A method according to claim 1, in which said step (b) is carried out in an atmosphere of nitrogen gas.

8. A method according to claim 1, in which in said step (b), the gas is sprayed to said suture needle (1).

EP 0 500 229 A1

# Fig.1

# Fig.2

6

# Fig.3

# Fig.4

# Fig.5

# Fig.6

**European Patent Office**

# EUROPEAN SEARCH REPORT

Application Number

EP 92 30 0917

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | WORLD PATENTS INDEX LATEST Section Ch, Week 8724, Derwent Publications Ltd., London, GB; Class A, AN 87-167959 & JP-A-62 101 236 (MATSUTANI SEISAKUSHO) 11 May 1987 * abstract * | 1,2 | B05D1/32 A61B17/06 C23C22/00 C09D5/00 |
| A,P | PATENT ABSTRACTS OF JAPAN vol. 015, no. 248 (C-0843)25 June 1991 & JP-A-3 080 869 ( MATSUTANI SEISAKUSHO ) 5 April 1991 * abstract * | 1,2,5 | |
| A,D | US-A-4 959 068 (BENDEL) | | |
| A,D | JP-U-63 026 309 (MATSUTANI SEISAKUSHO) | | |

TECHNICAL FIELDS SEARCHED (Int. Cl.5)

A61B
B05D

The present search report has been drawn up for all claims

| Place of search | Date of completion of the search | Examiner |
|---|---|---|
| THE HAGUE | 02 JUNE 1992 | KLEIN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)